# EUROPÄISCHE PATENTANMELDUNG

(11) **EP 1 174 442 A1**
(43) Veröffentlichungstag der Anmeldung: **23.01.2002**
(21) Anmeldenummer: 01116469.6
(22) Anmeldetag: 07.07.2001
(51) Int. Cl.: C08F 4/642, C08F 10/00, C07F 7/00

(54) **Komplexverbindungen und ihre Verwendung zur Polymerisation von Olefinen**

(30) Priorität: 20.07.2000 DE 10035700
(71) Anmelder: BASF AKTIENGESELLSCHAFT, 67056 Ludwigshafen (DE)
(72) Erfinder: Kristen, Marc Oliver, Dr., 67117 Limburgerhof (DE); Bildstein, Benno, Prof. Dr., 6020 Innsbruck (AT); Amort, Christoph, 39030 Rodeneck (IT); Malaun, Michael, Dr., 6473 Wenns (AT)

(57) **Zusammenfassung**

Komplexverbindungen der allgemeinen Formel I a und I b 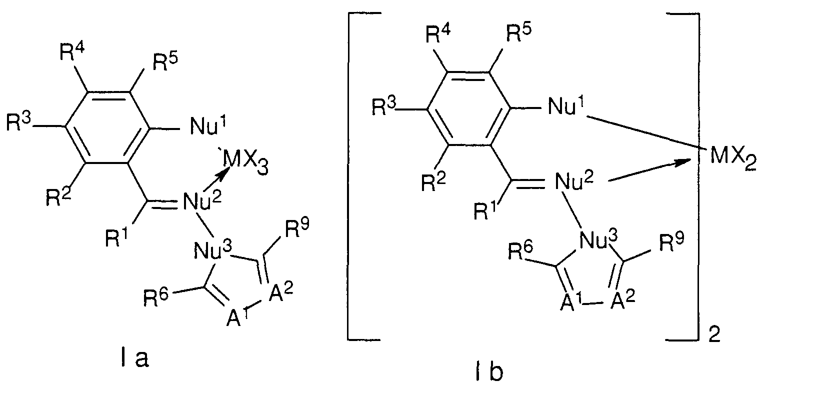 mit M = Ti, Zr, Hf oder V, Verfahren zur Herstellung der Metall-komplexe sowie die Verwendung der so erhältlichen Komplexe zur Polymerisation und Copolymerisation von Olefinen, beispielsweise in Suspensionspolymerisationsverfahren, Gasphasenpolymerisationsverfahren und Massepolymerisationsverfahren.

## Beschreibung

Die vorliegende Erfindung betrifftKomplexverbindungen der allgemeinen Formel I a und b, 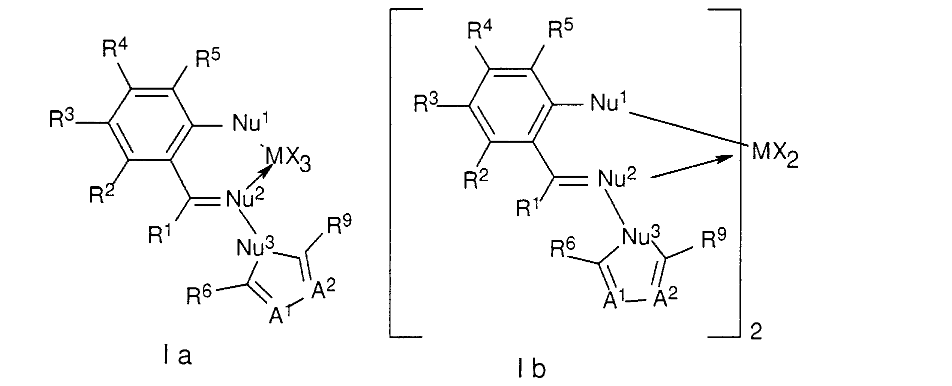 bei denen die Variablen wie folgt definiert sind:
- Nu¹: ausgewählt aus O, S oder Se;
- Nu²,Nu³: ausgewählt aus N oder P,
- M: ausgewählt aus Ti, Zr, Hf oder V, bevorzugt Ti oder Zr
- X: gleich oder verschieden und ausgewählt aus Halogen, C₁-C₈-Alkyl, C₃-C₁₂-Cycloalkyl, C₇-C₁₃-Aralkyl oder C₆-C₁₄-Aryl,
- A¹: N oder C-R⁷ oder Si-R⁷,
- A²: N oder C-R⁸ oder Si-R⁸,
- R¹ bis R⁹: gleich oder verschieden und ausgewählt aus Wasserstoff,
C₁-C₈-Alkyl, substituiert oder unsubstituiert,
C₂-C₈-Alkenyl, substituiert oder unsubstituiert, mit ein bis 4 isolierten oder konjugierten Doppelbindungen;
C₃-C₁₂-Cycloalkyl, substituiert oder unsubstituiert,
C₇-C₁₃-Aralkyl,
C₆-C₁₄-Aryl, unsubstituiert oder einfach oder mehrfach gleich oder verschieden substituiert mit
C₁-C₈-Alkyl, substituiert oder unsubstituiert,
C₃-C₁₂-Cycloalkyl,
C₇-C₁₃-Aralkyl,
C₆-C₁₄-Aryl,
Halogen,
C₁-C₆-Alkoxy, substituiert oder unsubstituiert,
C₆-C₁₄-Aryloxy,
SiR¹⁰R¹¹R¹² oder O-SiR¹⁰R¹¹R¹²;
fünf- bis sechsgliedrigen stickstoffhaltigen Heteroarylresten, unsubstituiert oder einfach oder mehrfach gleich oder verschieden substituiert mit
C₁-C₈-Alkyl, substituiert oder unsubstituiert,
C₃-C₁₂-Cycloalkyl,
C₇-C₁₃-Aralkyl,
C₆-C₁₄-Aryl,
Halogen,
C₁-C₆-Alkoxy,
C₆-C₁₄-Aryloxy,
SiR¹⁰R¹¹R¹² oder O-SiR¹⁰R¹¹R¹².
wobei räumlich benachbarte Reste R¹ bis R⁹ miteinander zu einem 5-bis 12-gliedrigen Ring verbunden sein können;
- R¹⁰ bis R¹²: gleich oder verschieden und ausgewählt werden aus Wasserstoff, C₁-C₈-Alkyl, C₃-C₁₂-Cycloalkyl, C₇-C₁₃-Aralkyl oder C₆-C₁₄-Aryl.

Weiterhin betrifft die vorliegende Erfindung ein Verfahren zur Herstellung der erfindungsgemäßen Komplexverbindungen sowie ein Verfahren zur Polymerisation oder Copolymerisation von Olefinen unter Verwendung einer Komplexverbindung der allgemeinen Formeln I a oder I b.

Schließlich betrifft die vorliegende Erfindung Trägerkatalysatoren und ein Verfahren zur Herstellung von Trägerkatalysatoren für die Polymerisation oder Copolymerisation von Olefinen unter Verwendung der erfindungsgemäßen Komplexverbindung der allgemeinen Formeln I a oder I b sowie ein Verfahren zur Polymerisation oder Copolymerisation von Olefinen unter Verwendung der erfindungsgemäßen Trägerkatalysatoren.

Polymere und Copolymere von Olefinen sind wirtschaftlich von großer Bedeutung, weil die Monomere in großen Mengen leicht zugänglich sind und weil sich die Polymere durch Variation des Herstellverfahrens oder der Verarbeitungsparameter in weiten Bereichen variieren lassen. Besondere Aufmerksamkeit beim Herstellverfahren gilt dabei dem verwendeten Katalysator. Neben Ziegler-Natta-Katalysatoren sind verschiedenartige Single-Site-Katalysatoren von wachsender Bedeutung, wobei als Zentralatome neben Zr wie beispielsweise in Metallocenkatalysatoren (H.-H. Brintzinger *et al*., *Angew*. *Chem*. **1995,** *107,* 1255) auch Ni oder Pd (WO 96/23010) oder Fe und Co (z.B. WO 98/27124) genauer untersucht worden sind. Die Komplexe von Ni, Pd, Fe und Co werden auch als Komplexe später Übergangsmetalle bezeichnet.

Metallocenkatalysatoren haben für den großtechnischen Einsatz Nachteile. Die am häufigsten verwendeten Metallocene, das sind Zirkonocene und Hafnocene, sind hydrolyseempfindlich. Außerdem sind die meisten Metallocene empfindlich gegenüber einer Vielzahl von Katalysatorgiften wie beispielsweise Alkoholen, Ethern oder CO, was eine sorgfältige Reinigung der Monomeren bedingt.

Während Ni- oder Pd-Komplexe (WO 96/23010) die Bildung hochverzweigter, kommerziell wenig interessanter Polymere katalysieren, führt die Verwendung von Fe- oder Co-Komplexen zur Bildung von hochlinearem Polyethylen mit sehr geringen Anteilen an Comonomer.

In EP-A 0 874 005 werden weitere polymerisationsaktive Komplexe offengelegt. Es handelt sich bei den Komplexen bevorzugt um Ti-Komplexe mit Salicylaldiminliganden. Auch sie tragen Phenylsubstituenten oder substituierte Phenylsubstituenten am Aldimin-Stickstoffatom (Seite 18-23) oder aber das Aldimin-Stickstoffatom ist in einen 6-gliedrigen Ring eingebaut (Seite 31-32). Sie erzeugen aber in der Regel niedermolekulare Polyethylene, die als Werkstoffe wenig geeignet sind.

Aufgrund der großen kommerziellen Bedeutung von Polyolefinen ist die Suche nach möglichst vielseitigen polymerisationsaktiven Komplexen auch weiterhin von großer Bedeutung.

Speziell bestand die Aufgabe,
- neue Komplexverbindungen bereitzustellen, die zur Polymerisation von Olefinen geeignet sind;
- ein Verfahren zur Herstellung der erfindungsgemäßen Komplexverbindungen bereitzustellen;
- ein Verfahren zur Polymerisation oder Copolymerisation von Olefinen unter Verwendung der erfindungsgemäßen Komplexverbindungen bereitzustellen;
- Trägerkatalysatoren für die Polymerisation von Olefinen sowie ein Verfahren zur Herstellung der erfindungsgemäßen Trägerkatalysatoren unter Verwendung der erfindungsgemäßen Komplexverbindungen bereitzustellen;
- mit den erfindungsgemäßen Trägerkatalysatoren Olefine zu polymerisieren und zu copolymerisieren.

Überraschend wurde nun gefunden, dass die Aufgabe mit Komplexverbindungen gelöst werden kann, die die eingangs definierten Strukturen der allgemeinen Formel Ia und Ib aufweisen.

In Formel I sind die Variablen wie folgt definiert:
- Nu¹: ausgewählt aus O, S und Se, wobei Sauerstoff bevorzugt ist;
- Nu²,Nu³: gleich oder verschieden und ausgewählt aus N oder P, wobei Nu² = Nu³ = Stickstoff besonders bevorzugt ist;
- M: ausgewählt aus Ti, Zr, Hf oder V, bevorzugt sind Ti oder Zr und besonders bevorzugt ist Ti;
- X: gleich oder verschieden und ausgewählt aus
- -: Halogen, wie Fluor, Chlor, Brom oder Iod, bevorzugt sind Chlor oder Brom und besonders bevorzugt ist Chlor;
- -: C₁-C₈-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, iso-Heptyl und n-Octyl; bevorzugt C₁-C₆-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, besonders bevorzugt C₁-C₄-Alkyl wie Methyl, Ethyl, n-Propyl, iso-Propyl, n-Butyl, iso-Butyl, sec.-Butyl und tert.-Butyl;
- -: C₃-C₁₂-Cycloalkyl, wie Cyclopropyl, Cyclobutyl, Cyclopentyl, Cyclohexyl, Cycloheptyl, Cyclooctyl, Cyclononyl,
Cyclodecyl, Cycloundecyl und Cyclododecyl; bevorzugt sind Cyclopentyl, Cyclohexyl und Cycloheptyl;
- -: C₇-C₁₃-Aralkyl bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, Neophyl (1-Methyl-1-phenylethyl), 1-Phenyl-butyl, 2-Phenyl-butyl, 3-Phenyl-butyl und 4-Phenyl-butyl, besonders bevorzugt Benzyl; oder
- -: C₆-C₁₄-Aryl, beispielsweise Phenyl, 1-Naphthyl, 2-Naphthyl, 1-Anthryl, 2-Anthryl, 9-Anthryl, 1-Phenanthryl, 2-Phenanthryl, 3-Phenanthryl, 4-Phenanthryl und 9-Phenanthryl, bevorzugt Phenyl, 1-Naphthyl und 2-Naphthyl, besonders bevorzugt Phenyl;
- A¹: N oder C-R⁷ oder Si-R⁷, wobei N oder C-R⁷ bevorzugt sind, besonders bevorzugt C-R⁷;
- A²: N oder C-R⁸ oder Si-R⁸, wobei N oder C-R⁸ bevorzugt sind, besonders bevorzugt C-R⁸;
- R¹ bis R⁹: sind gleich oder verschieden und ausgewählt aus
- -: Wasserstoff,
- -: C₁-C₈-Alkylgruppen, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, iso-Heptyl und n-Octyl; bevorzugt C₁-C₆-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, besonders bevorzugt C₁-C₄-Alkyl wie Methyl, Ethyl, n-Propyl, iso-Propyl, nButyl, iso-Butyl, sec.-Butyl und tert.-Butyl;
- -: Unter den substituierten C₁-C₈-Alkylgruppen seien beispielhaft genannt: ein- oder mehrfach halogenierte C₁-C₈-Alkylgruppen wie Fluormethyl, Difluormethyl, Trifluormethyl, Chlormethyl, Dichlormethyl, Trichlormethyl, Brommethyl, Dibrommethyl, Tribrommethyl, Pentafluorethyl, Perfluorpropyl und Perfluorbutyl, besonders bevorzugt sind Fluormethyl, Difluormethyl, Trifluormethyl und Perfluorbutyl;
- -: C₂-C₈-Alkenyl mit ein bis 4 isolierten oder konjugierten Doppelbindungen, beispielsweise Vinyl, 1-Allyl, 3-Allyl, ω-Butenyl, ω-Pentenyl, ω-Hexenyl, 1-cis-Buta-1,3-dienyl oder 1-cis -Hexa-1,5-dienyl.
- -: Unter den substituierten C₂-C₈-Alkenylgruppen seien beispielhaft genannt: Isopropenyl, 1-Isoprenyl, α-Styryl, β-Styryl, 1-cis-1,2-Phenylethenyl oder 1-trans-1,2-Phenylethenyl.
- -: C₃-C₁₂-Cycloalkyl wie Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl und Cycloheptyl;
- -: unter den substituierten Cycloalkylgruppen seien beispielhaft genannt:
2-Methylcyclopentyl, 3-Methylcyclopentyl,
cis-2,4-Dimethylcyclopentyl, trans-2,4-Dimethylcyclopentyl, cis-2,5-Dimethylcyclopentyl, trans-2,5-Dimethylcyclopentyl, 2,2,5,5-Tetramethylcyclopentyl, 2-Methylcyclohexyl, 3-Methylcyclohexyl, 4-Methylcyclohexyl, cis-2,6-Dimethylcyclohexyl, trans-2,6-Dimethylcyclohexyl, cis-2,6-Diisopropylcyclohexyl, trans-2,6-Diisopropylcyclohexyl, 2,2,6,6-Tetramethylcyclohexyl, 2-Methoxycyclopentyl, 2-Methoxycyclohexyl, 3-Methoxycyclopentyl, 3-Methoxycyclohexyl, 2-Chlorcyclopentyl, 3-Chlorcyclopentyl, 2,4-Dichlorcyclopentyl, 2,2,4,4-Tetrachlorcyclopentyl, 2-Chlorcyclohexyl, 3-Chlorcyclohexyl, 4-Chlorcyclohexyl, 2,5-Dichlorcyclohexyl, 2,2,6,6-Tetrachlorcyclohexyl,
2-Thiomethylcyclopentyl, 2-Thiomethylcyclohexyl, 3-Thiomethylcyclopentyl, 3-Thiomethylcyclohexyl und weitere Derivate;
- -: C₇-C₁₃-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, Neophyl (1-Methyl-1-phenylethyl), 1-Phenyl-butyl, 2-Phenyl-butyl, 3-Phenyl-butyl und 4-Phenyl-butyl, besonders bevorzugt Benzyl;
- -: C₆-C₁₄-Aryl, beispielsweise Phenyl, 1-Naphthyl, 2-Naphthyl, 1-Anthryl, 2-Anthryl, 9-Anthryl, 1-Phenanthryl, 2-Phenanthryl, 3-Phenanthryl, 4-Phenanthryl und 9-Phenanthryl, bevorzugt Phenyl, 1-Naphthyl und 2-Naphthyl, besonders bevorzugt Phenyl;
- -: C₆-C₁₄-Aryl wie Phenyl, 1-Naphthyl, 2-Naphthyl, 1-Anthryl, 2-Anthryl, 9-Anthryl, 1-Phenanthryl, 2-Phenanthryl, 3-Phenanthryl, 4-Phenanthryl und 9-Phenanthryl, gleich oder verschieden substituiert durch eine oder mehrere
- -: C₁-C₈-Alkylgruppen, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, iso-Heptyl und n-Octyl; bevorzugt C₁-C₆-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, besonders bevorzugt C₁-C₄-Alkyl wie Methyl, Ethyl, n-Propyl, iso-Propyl, n-Butyl, iso-Butyl, sec.-Butyl und tert.-Butyl;
- -: Unter den substituierten C₁-C₈-Alkylgruppen seien beispielhaft genannt: ein- oder mehrfach halogenierte C₁-C₈-Alkylgruppen wie Fluormethyl, Difluormethyl, Trifluormethyl, Chlormethyl, Dichlormethyl, Trichlormethyl, Brommethyl, Dibrommethyl, Tribrommethyl, Pentafluorethyl, Perfluorpropyl und Perfluorbutyl, besonders bevorzugt sind Fluormethyl, Difluormethyl, Trifluormethyl und Perfluorbutyl;
- -: C₃-C₁₂-Cycloalkyl wie Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl und Cycloheptyl;
- -: C₇-C₁₃-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, Neophyl (1-Methyl-1-phenylethyl), 1-Phenyl-butyl, 2-Phenyl-butyl, 3-Phenyl-butyl und 4-Phenyl-butyl, besonders bevorzugt Benzyl;
- -: C₆-C₁₄-Aryl, beispielsweise Phenyl, 1-Naphthyl, 2-Naphthyl, 1-Anthryl, 2-Anthryl, 9-Anthryl, 1-Phenanthryl, 2-Phenanthryl, 3-Phenanthryl, 4-Phenanthryl und 9-Phenanthryl, bevorzugt Phenyl, 1-Naphthyl und 2-Naphthyl, besonders bevorzugt Phenyl;
- -: Halogen, beispielsweise Fluor, Chlor, Brom oder Iod, besonders bevorzugt Fluor oder Chlor;
- -: C₁-C₆-Alkoxygruppen wie Methoxy, Ethoxy, n-Propoxy, iso-Propoxy, n-Butoxy, iso-Butoxy, sec.-Butoxy, tert.-Butoxy, n-Pentoxy, iso-Pentoxy, n-Hexoxy und iso-Hexoxy, besonders bevorzugt Methoxy, Ethoxy, n-Propoxy und n-Butoxy;
- -: C₆-C₁₄-Aryloxygruppen wie Phenoxy, ortho-Kresyloxy, meta-Kresyloxy, para-Kresyloxy, α-Naphthoxy, β-Naphthoxy oder 9-Anthryloxy;
- -: Silylgruppen SiR¹⁰R¹¹R¹², wobei R¹⁰ bis R¹² unabhängig voneinander aus Wasserstoff, C₁-C₈-Alkylgruppen, Benzylresten und C₆-C₁₄-Arylgruppen ausgewählt sind; bevorzugt sind die Trimethylsilyl-, Triethylsilyl-, Triisopropylsilyl-, Diethylisopropylsilyl-, Dimethylthexylsilyl-, tert.-Butyldimethylsilyl-, tert.-Butyldiphenylsilyl-, Tribenzylsilyl-, Triphenylsilyl- und die Tri-para-xylylsilylgruppe; besonders bevorzugt sind die Trimethylsilylgruppe und die tert.-Butyldimethylsilylgruppe;
- -: Silyloxygruppen OSiR¹⁰R¹¹R¹², wobei R¹⁰ bis R¹² unabhängig voneinander aus Wasserstoff, C₁-C₈-Alkylgruppen, Benzylresten und C₆-C₁₄-Arylgruppen ausgewählt sind; bevorzugt sind die Trimethylsilyloxy-, Triethylsilyloxy-, Triisopropylsilyloxy-, Diethylisopropylsilyloxy-, Dimethylthexylsilyloxy-, tert.-Butyldimethylsilyloxy-, tert.-Butyldiphenylsilyloxy-, Tribenzylsilyloxy-, Triphenylsilyloxy- und die Tri*-para*-xylylsilyloxygruppe; besonders bevorzugt sind die Trimethylsilyloxygruppe und die tert.-Butyldimethylsilyloxygruppe;
- -: fünf- bis sechsgliedrigen stickstoffhaltigen Heteroarylresten wie beispielsweise N-Pyrrolyl, Pyrrol-2-yl, Pyrrol-3-yl, *N*-Imidazolyl, 2-Imidazolyl, 4-Imidazolyl, 1,2,4-Triazol-3-yl, 1,2,4-Triazol-4-yl, 2-Pyridyl, 3-Pyridyl, 4-Pyridyl, 3-Pyridazinyl, 4-Pyridazinyl, 2-Pyrimidinyl, 4-Pyrimidinyl, 5-Pyrimidinyl, N-Indolyl und N-Carbazolyl;
- -: fünf- bis sechsgliedrigen stickstoffhaltigen Heteroarylresten wie beispielsweise *N*-Pyrrolyl, Pyrrol-2-yl, Pyrrol-3-yl, *N*-Imidazolyl, 2-Imidazolyl, 4-Imidazolyl, 1,2,4-Triazol-3-yl, 1,2,4-Triazol-4-yl, 2-Pyridyl, 3-Pyridyl, 4-Pyridyl, 3-Pyridazinyl, 4-Pyridazinyl, 2-Pyrimidinyl, 4-Pyrimidinyl, 5-Pyrimidinyl, N-Indolyl und N-Carbazolyl, gleich oder verschieden einfach oder mehrfach substituiert mit
- -: C₁-C₈-Alkylgruppen, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, iso-Heptyl und n-Octyl; bevorzugt C₁-C₆-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, besonders bevorzugt C₁-C₄-Alkyl wie Methyl, Ethyl, n-Propyl, iso-Propyl, n-Butyl, iso-Butyl, sec.-Butyl und tert.-Butyl;
- -: Unter den substituierten C₁-C₈-Alkylgruppen seien beispielhaft genannt: ein- oder mehrfach halogenierte C₁-C₈-Alkylgruppen wie Fluormethyl, Difluormethyl, Trifluormethyl, Chlormethyl, Dichlormethyl, Trichlormethyl, Brommethyl, Dibrommethyl, Tribrommethyl, Pentafluorethyl, Perfluorpropyl und Perfluorbutyl, besonders bevorzugt sind Fluormethyl, Difluormethyl, Trifluormethyl und Perfluorbutyl;
- -: C₃-C₁₂-Cycloalkyl wie Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl und Cycloheptyl;
- -: C₇-C₁₃-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, Neophyl (1-Methyl-1-phenylethyl), 1-Phenyl-butyl, 2-Phenyl-butyl, 3-Phenyl-butyl und 4-Phenyl-butyl, besonders bevorzugt Benzyl;
- -: C₆-C₁₄-Aryl, beispielsweise Phenyl, 1-Naphthyl, 2-Naphthyl, 1-Anthryl, 2-Anthryl, 9-Anthryl, 1-Phenanthryl, 2-Phenanthryl, 3-Phenanthryl, 4-Phenanthryl und 9-Phenanthryl, bevorzugt Phenyl, 1-Naphthyl und 2-Naphthyl, besonders bevorzugt Phenyl; Halogen, beispielsweise Fluor, Chlor, Brom oder Iod, besonders bevorzugt Fluor oder Chlor;
- -: C₁-C₆-Alkoxygruppen wie Methoxy, Ethoxy, n-Propoxy, iso-Propoxy, n-Butoxy, iso-Butoxy, sec.-Butoxy, tert.-Butoxy, n-Pentoxy, iso-Pentoxy, n-Hexoxy und iso-Hexoxy, besonders bevorzugt Methoxy, Ethoxy, n-Propoxy und n-Butoxy; C₆-C₁₄-Aryloxygruppen wie Phenoxy, *ortho*-Kresyloxy, *meta-* Kresyloxy, *para*-Kresyloxy, α-Naphthoxy, β-Naphthoxy oder 9-Anthryloxy;
- -: Silylgruppen SiR¹⁰R¹¹R¹², wobei R¹⁰ bis R¹² unabhängig voneinander aus Wasserstoff, C₁-C₈-Alkylgruppen, Benzylresten und C₆-C₁₄-Arylgruppen ausgewählt sind; bevorzugt sind die Trimethylsilyl-, Triethylsilyl-, Triisopropylsilyl-, Diethylisopropylsilyl-, Dimethylthexylsilyl-, tert.-Butyldimethylsilyl-, tert.-Butyldiphenylsilyl-, Tribenzylsilyl-, Triphenylsilyl- und die Tri-para-xylylsilylgruppe; besonders bevorzugt sind die Trimethylsilylgruppe und die tert.-Butyldimethylsilylgruppe;
- -: Silyloxygruppen OSiR¹⁰R¹¹R¹², wobei R¹⁰ bis R¹² unabhängig voneinander aus Wasserstoff, C₁-C₈-Alkylgruppen, Benzylresten und C₆-C₁₄-Arylgruppen ausgewählt sind; bevorzugt sind die Trimethylsilyloxy-, Triethylsilyloxy-, Triisopropylsilyloxy-, Diethylisopropylsilyloxy-, Dimethylthexylsilyloxy-, tert.-Butyldimethylsilyloxy-, tert.-Butyldiphenylsilyloxy-, Tribenzylsilyloxy-, Triphenylsilyloxyund die Tri-para-xylylsilyloxygruppe; besonders bevorzugt sind die Trimethylsilyloxygruppe und die tert.-Butyldimethylsilyloxygruppe;

In einer besonders bevorzugten Ausführungsform sind R¹, R² und R⁴ Wasserstoff. In einer ebenfalls besonders bevorzugten Ausführungsform sind R⁶ und R⁹ C₁-C₈-Alkyl, verzweigt oder unverzweigt. R³ und R⁵ sind besonders bevorzugt unabhängig voneinander Wasserstoff oder C₁-C₈-Alkyl, verzweigt oder unverzweigt.

In einer besonderen Ausführungsform können benachbarte Reste R¹ bis R⁹ miteinander zu einem 5 bis 12-gliedrigen Ring verbunden sein. Beispielsweise können R⁶ und R⁷ zusammen sein: -(CH₂)₃-(Trimethylen), -(CH₂)₄- (Tetramethylen), -(CH₂)₅- (Pentamethylen), -(CH₂)₆- (Hexamethylen), -CH₂-CH=CH-, -CH₂-CH=CH-CH₂-, -CH=CH-CH=CH-, -O-CH₂-O-, -O-CHMe-O-, -O-CH-(C₆H₅)-O-, -O-CH₂-CH₂-O-, -O-CMe₂-O-, -NMe-CH₂-CH₂-NMe-, -NMe-CH₂-NMe- oder -O-SiMe₂-O- mit Me = CH₃. In einem bevorzugten Beispiel bilden R⁶ und R⁷ zusammen eine 1,3-Butadien-1,4-diyl-Einheit, die ihrerseits mit C₁-C₈-Alkyl ein oder mehrfach substituiert sein kann. In einem weiteren bevorzugten Beispiel bilden R⁶ und R⁷ sowie R⁸ und R⁹ jeweils paarweise zusammen eine 1,3-Butadien-1,4-diyl-Einheit, die ihrerseits mit C₁-C₈-Alkyl ein oder mehrfach substituiert sein können.

Zur Synthese der erfindungsgemäßen Komplexverbindungen der allgemeinen Formel I geht man im Allgemeinen von einem Liganden der allgemeinen Formel II aus, 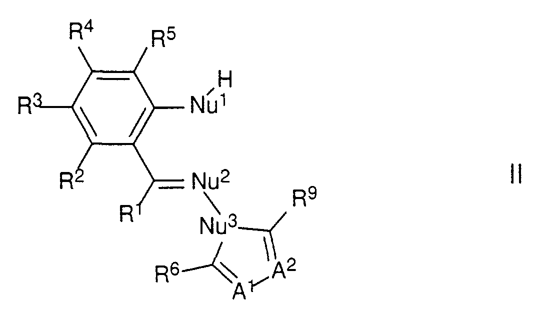 in dem die Variablen wie vorstehend definiert sind.

Die Liganden der allgemeinen Formel II werden zunächst mit Hilfe einer Base deprotoniert und anschließend mit Metallverbindungen der allgemeinen Formel MX₄ umgesetzt.

Als Base können die in der Metallorganischen Chemie gängigen Metallalkyle verwendet werden wie beispielsweise Methyllithium, Ethyllithium, n-Butyllithium, sec-Butyllithium, tert.-Butyllithium oder Hexyllithium, weiterhin Grignard-Verbindungen wie beispielsweise Ethylmagnesiumbromid, weiterhin Lithiumamid, Natriumamid, Kaliumamid, Kaliumhydrid oder Lithiumdiisopropylamid ("LDA"). Als Lösemittel haben sich hochsiedende Lösemittel wie Toluol, ortho-Xylol, meta-Xylol, para-Xylol, Ethylbenzol oder Mischungen derselben als geeignet erwiesen, des Weiteren nichtcyclische oder cyclische Ether wie 1,2-Dimethoxyethan, Tetrahydrofuran oder Diethylether.

Diese Deprotonierung ist im Allgemeinen nach einigen Stunden beendet, sinnvoll ist eine Reaktionsdauer von 2 bis 10 Stunden, bevorzugt sind 3 bis 5 Stunden. Die Temperaturbedingungen sind im Allgemeinen unkritisch, eine Durchführung bei Temperaturen von -90°C bis -20°C hat sich als bevorzugt erwiesen.

Der deprotonierte Ligand und die Metallverbindung der allgemeinen Formel MX₄ werden anschließend miteinander umgesetzt.

Dabei kann MX₄ optional durch 1 bis 2 gleiche oder verschiedene Neutralliganden stabilisiert werden. Als Neutralliganden bieten sich die gängigen Liganden der Komplexchemie an, wie beispielsweise cyclische und nichtcyclische Ether, Amine, Diamine, Nitrile, Isonitrile oder Phosphine. Besonders bevorzugt sind Diethylether, Tetrahydrofuran, 1,2-Dimethoxyethan, Tetramethylethylendiamin, Acetonitril oder Triphenylphosphan.

Die Bedingungen für die Umsetzung sind an sich unkritisch; üblicherweise mischt man den deprotonierten Liganden I und MX₄ miteinander in einem geeigneten Lösemittel wie Benzol, Toluol, Ethylbenzol, ortho-Xylol, meta-Xylol oder para-Xylol, Chlorbenzol, Cyclohexan, Methylenchlorid oder einem Gemisch derselben. Als Temperaturbereich kommen -100°C bis +150°C in Frage, bevorzugt -78°C bis +100°C. Dabei sollte die Reaktionstemperatur den Schmelzpunkt des Lösemittels nicht unterschreiten; Temperaturen oberhalb des Siedepunkts des betreffenden Lösemittels lassen sich in Autoklavenversuchen verwirklichen. Wichtig ist, dass man die Umsetzung unter Ausschluss von Sauerstoff und Feuchtigkeit durchführt.

Als Molverhältnisse zwischen Ligand und M sind solche im Bereich von 5:1 bis 1:5 geeignet. Da jedoch die Liganden der allgemeinen Formel II die aufwändiger zugänglichen Reaktionspartner sind, sind Molverhältnisse Ligand : M im Bereich von 1:1 bis 1:3 bevorzugt, besonders bevorzugt sind stöchiometrische Mengen.

Wünscht man jedoch Verbindungen der allgemeinen Formel I b zu erhalten, so sind Molverhältnisse Ligand : M von 2:1 bis 4:1 bevorzugt.

Die Reinigung der erfindungsgemäßen Komplexverbindungen der allgemeinen Formel Ia und Ib gelingt durch die in der metallorganischen Chemie üblichen Methoden, wobei Kristallisation und Ausfällen besonders bevorzugt ist, weiterhin sind Filtrationen über Filterhilfsmittel wie beispielsweise Celite® geeignet.

Die Herstellung der Liganden der allgemeinen Formel II gelingt durch Kondensation einer Carbonylverbindung der allgemeinen Formel III 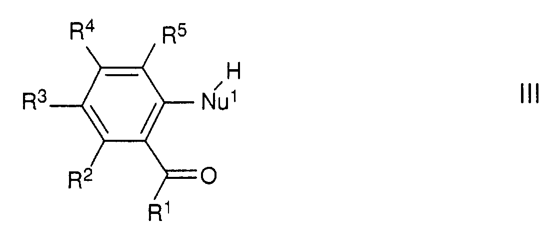 mit einer Verbindung der allgemeinen Formel IV, 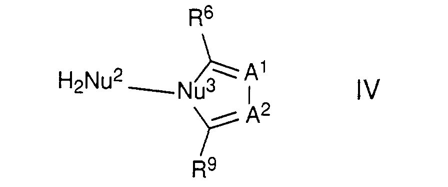 in denen die Variablen wie eingangs definiert sind.

Die Synthese wird bei Temperaturen von -78°C bis + 150°C durchgeführt, bevorzugt bei -10°C bis +75°C. Als Katalysator wird eine Lewis-Säure oder eine Brønsted-Säure zugegeben. Als Lewis-Säuren haben sich insbesondere Aluminiumalkyle wie Al(CH₃)₃, Al(C₂H₅)₃ oder BF₃ als wirksam erwiesen. Als Brønsted-Säuren lassen sich beispielsweise Schwefelsäure, Phosphorsäure, HF, Toluolsulfonsäure oder Amidosulfonsäure verwenden. Die Reaktionsdauer beträgt 1 bis 48 Stunden, bevorzugt 12 bis 24 Stunden. Als Lösemittel haben sich aprotische Medien als bevorzugt erwiesen, insbesondere Toluol und Benzol, wenn Aluminiumalkyle oder BF₃ als Katalysatoren verwendet wurden, ansonsten lassen sich auch Alkohole wie Methanol, Ethanol oder Mischungen derselben verwenden. Es hat sich - insbesondere bei der Verwendung einer Brønsted-Säure als Katalysator - als wirksam erwiesen, mit Hilfe eines Wasserabscheiders das gebildete Wasser azeotrop abzudestillieren.

Verbindungen der allgemeinen Formel III und ein Verfahren zu ihrer Herstellung sind in WO 98/42664 beschrieben.

Die Synthese der Verbindungen der allgemeinen Formel IV gelingt nach dem in DE-A 199 44 993, publiziert am ..., beschriebenen Verfahren, indem man zunächst eine geeignete 1,4-Dicarbonylverbindung der allgemeinen Formel V 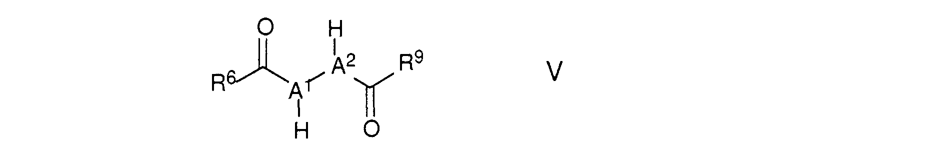 mit einem Äquivalent Acetylhydrazin oder Benzoylhydrazin in Anwesenheit einer katalytischen Menge Säure, bevorzugt para-Toluolsulfonsäure, in einem inerten Lösemittel, bevorzugt Toluol, zu einem N-Acetyl- oder N-Benzoyl-geschützten Derivat von IV umsetzt und anschließend mit einem Überschuß an Base, vorzugsweise KOH, in einem hochsiedenden organischen Lösemittel wie beispielsweise Ethylenglykol verseift.

Damit die erfindungsgemäßen Komplexverbindungen der allgemeinen Formeln I a oder I b katalytisch aktiv sind, müssen sie aktiviert werden. Geeignete Aktivatoren sind ausgewählte Aluminium- oder Bor-Verbindungen mit elektronenziehenden Resten (z.B. Trispentafluorphenylboran, Trispentafluorphenylaluminium, *N,N*-Dimethylanilinium-tetrakis-pentafluorphenylborat, Tri-n-butylammoniumtetrakis-pentafluorphenylborat, *N,N*-Dimethylanilinium-tetrakis-(3,5-bisperfluormethyl)-phenylborat, Tri-n-butylammonium-tetrakis-(3,5-bisperfluormethyl)-phenylborat sowie Tritylium-tetrakispentafluorphenylborat). Bevorzugt sind Dimethylaniliniumtetrakis-pentafluorphenylborat, Tritylium-tetrakispentafluorphenylborat sowie Trispentafluorphenylboran.

Verwendet man Bor- oder Aluminiumverbindungen als Aktivatoren für die Komplexe der allgemeinen Formel I a und I b, so setzt man sie im Allgemeinen in einem molaren Verhältnis von 1 : 10 bis 10 : 1, bezogen auf M, ein; bevorzugt 1 : 2 bis 5 : 1 und besonders bevorzugt 1 : 1,5 bis 1,5 : 1.

Eine andere geeignete Klasse von Aktivatoren sind Aluminoxane. Die Struktur der Aluminoxane ist nicht genau bekannt. Es handelt sich bei ihnen um Produkte, die durch vorsichtige partielle Hydrolyse von Aluminiumalkylen erhalten werden (s. DE-A 30 07 725). Diese Produkte liegen nicht rein vor, sondern als Gemische von offenkettigen und cyclischen Strukturen des Typs VIa und VIb. Diese Gemische liegen vermutlich in einem dynamischen Gleichgewicht zueinander vor.

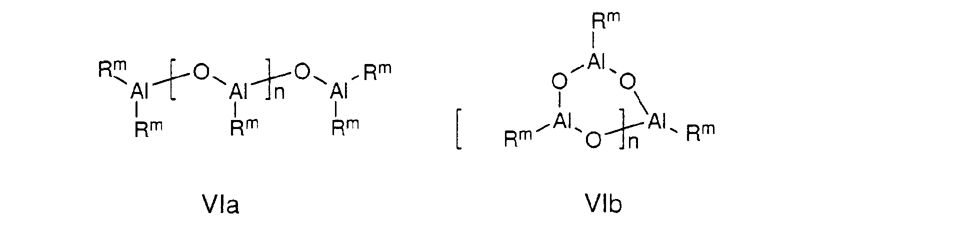

In Formel VIa und VIb sind die Reste R^{m} unabhängig voneinander
- -: -C₁-C₁₂-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, iso-Heptyl, n-Octyl, n-Nonyl, n-Decyl, und n-Dodecyl; bevorzugt C₁-C₆-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, besonders bevorzugt ist Methyl;
- -: C₃-C₁₂-Cycloalkyl wie Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl und Cycloheptyl;
- -: C₇- bis C₂₀-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, oder
- -: C₆-C₁₄-Aryl wie Phenyl, 1-Naphthyl, 2-Naphthyl, 1-Anthryl, 2-Anthryl, 9-Anthryl, 1-Phenanthryl, 2-Phenanthryl, 3-Phenanthryl, 4-Phenanthryl und 9-Phenanthryl, bevorzugt Phenyl, 1-Naphthyl und 2-Naphthyl, besonders bevorzugt Phenyl; und
- n: ist eine ganze Zahl von 0 bis 40, bevorzugt von 1 bis 25 und besonders bevorzugt von 2 bis 22.

In der Literatur werden auch käfigartige Strukturen für Aluminoxane diskutiert (Y. Koide, S.G. Bott, A.R. Barron *Organometallics* **1996,** *15,* 2213-26; A.R. Barron *Macromol. Symp.* **1995,** 97, 15-25). Unabhängig davon, wie die Struktur der Aluminoxane tatsächlich aussieht, sind sie als Aktivatoren der erfindungsgemäßen Metallkomplexe der allgemeinen Formeln I a oder I b geeignet.

Gemische verschiedener Aluminoxane sind in den Fällen besonders bevorzugte Aktivatoren, in denen in einer Lösung eines Paraffins, beispielsweise n-Heptan oder Isododekan, polymerisiert wird. Eine besonders bevorzugtes Gemisch ist das kommerziell bei der Firma Witco GmbH erhältliche CoMAO mit einer Formel von [(CH₃)_{0,9}(iso-C₄H₉)_{0,1}AlO]ₙ.

Um die Komplexe der allgemeinen Formel I a oder I b mit Aluminoxanen zu aktivieren, ist im Allgemeinen ein Überschuß von Aluminoxan, bezogen auf M, notwendig. Sinnvolle Molverhältnisse M : Al liegen im Bereich von 1 : 10 bis 1 : 10.000, bevorzugt 1 : 50 bis 1 : 1000 und besonders bevorzugt 1 : 100 bis 1 : 500.

Die gewählte Komplexverbindung der allgemeinen Formeln I a oder I b und der Aktivator bilden zusammen ein Katalysatorsystem.

Durch Zugabe von weiterem Aluminiumalkyl der allgemeinen Formel Al(R^{m})₃ oder Aluminoxanen kann die Aktivität des erfindungsgemäßen Katalysatorsystems erhöht werden; Aluminiumalkyle der allgemeinen Formel Al(R^{m})₃ oder Aluminoxane können auch als Molmassenregler wirken. Ein weiterer effektiver Molmassenregler ist Wasserstoff. Besonders gut kann man die Molmasse durch die Reaktionstemperatur und den Druck regeln. Für den Fall, dass die Verwendung einer Bor-Verbindung wie oben beschrieben gewünscht ist, ist die Zugabe eines Aluminiumalkyls der allgemeinen Formel Al(R^{m})₃ besonders bevorzugt.

Es wurde gefunden, dass die erfindungsgemäßen Komplexverbindungen der allgemeinen Formel I a oder b geeignet sind, um Olefine zu polymerisieren. Besonders gut polymerisieren und copolymerisieren sie Ethylen und Propylen zu hochmolekularen Polymeren. Komplexe der allgemeinen Formel I b sind chiral, sie liefern bei der Polymerisation isotaktisches Polypropylen.

Druck- und Temperaturbedingungen während der Polymerisation können in weiten Grenzen gewählt werden. Als Druck hat sich ein Bereich von 0,5 bar bis 4000 bar als geeignet erwiesen, bevorzugt sind 10 bis 75 bar oder Hochdruckbedingungen von 500 bis 2500 bar. Als Temperatur hat sich ein Bereich von 0 bis 120°C als geeignet erwiesen, bevorzugt sind 40 bis 100°C und besonders bevorzugt 50 bis 85°C.

Als Monomer sind die folgenden Olefine zu nennen: Ethylen, Propylen, 1-Buten, 1-Penten, 1-Hexen, 1-Octen, 1-Decen oder 1-Undecen, wobei Propylen und Ethylen bevorzugt und Ethylen besonders bevorzugt ist.

Als Comonomere sind α-Olefine geeignet, wie beispielsweise 0,1 bis 20 mol-% 1-Buten, 1-Penten, 1-Hexen, 4-Methyl-1-Penten, 1-Octen, 1-Decen oder 1-Undecen. Aber auch Isobuten und Styrol sind geeignete Comonomere, des weiteren interne Olefine wie beispielsweise Cyclopenten, Cyclohexen, Norbornen und Norbornadien.

Als Lösemittel haben sich Toluol, ortho-Xylol, meta-Xylol, para-Xylol oder Ethylbenzol als geeignet erwiesen sowie Mischungen derselben, weiterhin - bei Hochdruckbedingungen - überkritisches Ethylen.

Die erfindungsgemäßen Komplexverbindungen der allgemeinen Formel I a oder b lassen sich bei der Polymerisation mit Wasserstoff regeln, d.h. durch Zugabe von Wasserstoff lässt sich das Molekulargewicht der durch das erfindungsgemäße Katalysatorsystem erhältlichen Polymere senken. Bei genügend Wasserstoffzugabe werden Wachse erhalten, wobei die erforderliche Wasserstoffkonzentration auch von der Art der verwendeten Polymerisationsanlage abhängt.

Damit die erfindungsgemäßen Komplexverbindungen in modernen Polymerisationsverfahren wie Suspensionsverfahren, Massepolymerisationsverfahren oder Gasphasenverfahren eingesetzt werden können, ist es notwendig, sie auf einem festen Träger zu immobilisieren. Andernfalls kann es zu Morphologieproblemen des Polymers (Brokken, Wandbeläge, Verstopfungen in Leitungen oder Wärmetauschern) kommen, die zum Abschalten der Anlage zwingen. Eine solche immobilisierte Komplexverbindung wird als Katalysator bezeichnet.

Es wurde gefunden, dass sich die erfindungsgemäßen Komplexverbindungen gut auf einem festen Träger abscheiden lassen. Als Trägermaterialien kommen z.B. poröse Metalloxide von Metallen der Gruppen 2 bis 14 oder Mischungen derselben in Frage, weiterhin Schichtsilikate und Zeolithe. Bevorzugte Beispiele für Metalloxide der Gruppen 2 bis 14 sind SiO₂, B₂O₃, Al₂O₃, MgO, CaO und ZnO. Bevorzugte Schichtsilikate sind Montmorrilonite oder Bentonite; als bevorzugter Zeolith wird MCM-41 eingesetzt.

Besonders bevorzugte Trägermaterialien sind sphärische Kieselgele und Alumosilikatgele der allgemeinen Formel SiO₂·a Al₂O₃, wobei a allgemein für eine Zahl im Bereich von 0 bis 2 steht, bevorzugt 0 bis 0,5. Derartige Kieselgele sind im Handel erhältlich, z.B. Silica Gel SG 332, Sylopol® 948 oder 952 oder S 2101 der Fa. W.R. Grace oder ES 70X der Fa. Crosfield.

Als Partikelgröße des Trägermaterials haben sich mittlere Teilchendurchmesser von 1 bis 300 µm bewährt, bevorzugt von 20 bis 80 µm, wobei der Teilchendurchmesser durch bekannte Methoden wie Siebmethoden bestimmt wird. Das Porenvolumen dieser Träger beträgt 1,0 bis 3,0 ml/g, bevorzugt von 1,6 bis 2,2 ml/g und besonders bevorzugt von 1,7 bis 1,9 ml/g. Die BET-Oberfläche beträgt 200 bis 750 m²/g, bevorzugt 250 bis 400 m²/g.

Um dem Trägermaterial anhaftende Verunreinigungen, insbesondere Feuchtigkeit, zu entfernen, können die Trägermaterialien vor der Dotierung ausgeheizt werden, wobei sich Temperaturen von 45 bis 1000°C eignen. Temperaturen von 100 bis 750°C sind für Kieselgele und andere Metalloxide besonders geeignet. Dieses Ausheizen kann über einen Zeitraum von 0,5 bis 24 Stunden erfolgen, wobei Ausheizzeiten von 1 bis 12 Stunden bevorzugt sind. Die Druckbedingungen sind vom gewählten Verfahren abhängig; das Ausheizen kann in einem Festbettverfahren, einem gerührten Kessel oder aber in einem Fließbettverfahren erfolgen. Ganz allgemein kann das Ausheizen bei Atmosphärendruck erfolgen. Vorteilhaft sind jedoch verminderte Drücke von 0,1 bis 500 mbar, besonders vorteilhaft ist ein Bereich von 1 bis 100 mbar und ganz besonders vorteilhaft ein Bereich von 2 bis 20 mbar. Für Fließbettverfahren hingegen empfiehlt es sich, bei leicht erhöhtem Druck zu arbeiten, wobei der Druck in einem Bereich von 1,01 bar bis 5 bar, bevorzugt 1,1 bis 1,5 bar gewählt wird.

Eine chemische Vorbehandlung des Trägermaterials mit einer Alkylverbindung wie Aluminiumalkyl, Lithiumalkyl oder einem Alumoxan ist ebenfalls möglich.

Für eine Polymerisation im Suspensionsverfahren werden solche Suspensionsmittel verwendet, in denen das gewünschte Polymer nicht oder nur in geringem Ausmaß löslich ist, weil andernfalls in den Anlagenteilen, in denen das Produkt vom Suspensionsmittel abgetrennt wird, Beläge des Produkts auftreten und zu wiederholten Abschaltungen und Reinigungsoperationen zwingen. Geeignete Suspensionsmittel sind gesättigte Kohlenwasserstoffe wie beispielsweise Propan, n-Butan, Isobutan, n-Pentan, Isopentan, n-Hexan, Isohexan und Cyclohexan, wobei Isobutan bevorzugt ist.

Druck- und Temperaturbedingungen während der Polymerisation können in weiten Grenzen gewählt werden. Als Druck hat sich ein Bereich von 0,5 bar bis 150 bar als geeignet erwiesen, bevorzugt sind 10 bis 75 bar. Als Temperatur hat sich ein Bereich von 0 bis 120°C als geeignet erwiesen, bevorzugt sind 40 bis 100°C.

Als Monomer sind die folgenden Olefine zu nennen: Ethylen, Propylen, 1-Buten, 1-Penten, 1-Hexen, 1-Octen, 1-Decen oder 1-Undecen.

Als Comonomere sind α-Olefine geeignet, wie beispielsweise 0,1 bis 20 mol-% 1-Buten, 1-Penten, 1-Hexen, 4-Methyl-1-Penten, 1-Octen, 1-Decen oder 1-Undecen. Aber auch Isobuten und Styrol sind geeignete Comonomere, des weiteren interne Olefine wie beispielsweise Cyclopenten, Cyclohexen, Norbornen und Norbornadien.

Die erfindungsgemäßen Katalysatoren haben sich weiterhin als Wasserstoff-regelbar erwiesen, d.h. durch Zugabe von Wasserstoff lässt sich das Molekulargewicht der durch die erfindungsgemäßen Katalysatoren erhältlichen Polymere senken. Bei genügend Wasserstoffzugabe werden Wachse erhalten, wobei die erforderliche Wasserstoffkonzentration auch von der Art der verwendeten Polymerisationsanlage abhängt. Bei Wasserstoffzugabe steigt die Aktivität der erfindungsgemäßen Katalysatoren.

Die erfindungsgemäßen Katalysatoren können auch gemeinsam mit einem oder mehreren anderen, an sich bekannten Polymerisationskatalysatoren verwendet werden. So können sie zusammen mit
- Ziegler-Natta-Katalysatoren,
- geträgerten Metallocenkatalysatoren der Übergangsmetalle der Gruppen 4 bis 6 des Periodensystems der Elemente,
- Katalysatoren der späten Übergangsmetalle (WO 96/23010),
- Fe- oder Co-Komplexen mit Pyridyldiiminliganden, wie sie in WO 98/27124 offenbart werden,
- oder auch Chromoxidkatalysatoren nach Phillips eingesetzt werden.

Dabei ist es einerseits möglich, verschiedene Katalysatoren miteinander zu mischen und gemeinsam zu dosieren oder cogeträgerte Komplexe auf einem gemeinsamen Träger zu verwenden oder auch verschiedene Katalysatoren getrennt an derselben oder an verschiedenen Stellen in das Polymerisationsgefäß zu dosieren.

Nachfolgende Arbeitsbeispiele erläutern die Erfindung.

### Allgemeine Vorbemerkungen:

Alle Arbeiten wurden unter Ausschluß von Luft und Feuchtigkeit unter Verwendung von Standard-Schlenk-Techniken hergestellt. Geräte und Chemikalien waren entsprechend vorbereitet. Die Polymerviskosität wurde nach ISO 1628-3 bestimmt.

### 1. Darstellung der erfindungsgemäßen Liganden:

Allgemeine Arbeitsvorschrift:
   Äquimolare Mengen der entsprechenden Salicylaldehyde und der Aminoazole wurden in möglichst wenig Methanol gelöst, mit 0,5 ml Ameisensäure versetzt und anschließend 12 Stunden bei Raumtemperatur gerührt. In den meisten Fällen konnte das Produkt durch Abfiltrieren des entstandenen Niederschlages und mehrmaliges Waschen mit Methanol analysenrein isoliert werden. Anschließend wurde das Reaktionsgemisch am Rotationsverdampfer eingeengt und anschließend säulenchromatographisch gereinigt (Kieselgel Merck 60®, Laufmittel Hexan/ Ether 3:2). Man erhielt Ligand 1 als hell gelbes Öl. 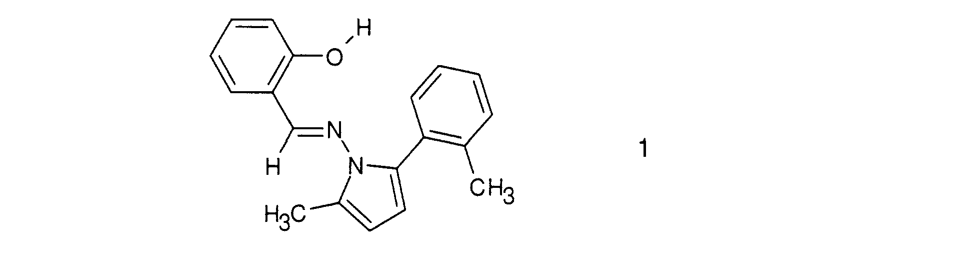
Analytische Daten:
   1: hell gelbes Öl, Ausbeute: 95% d. Theorie, C₁₉H₁₈N₂O. MS(EI): m/z 290 (M⁺, 100%), 170 (M⁺-HOC₆H₄CN, 51%). IR(KBr): 3062 w, 2923 w, 1622 s, 1607 s, 1572 w, 1522 w, 1489 m, 1472 w, 1458 w, 1395 m, 1356 w, 1290 s, 1283 s, 1269 s, 1186 w, 1153 m, 1122 w, 1034 w, 958 w, 820 w, 754 s, 725 m, 607 w, 478 w cm⁻¹. ¹H NMR (CDCl₃, δ) : 2.14 (3H, s, CH₃); 2.40 (3H, s, CH₃); 6.02 (1H, m *J* = 3.78 Hz, Pyrrol); 6.11 (1H, d *J* = 3.78 Hz, Pyrrol); 6.72-7.36 (8H, m, Phenyl); 7.86 (1H, s, Imin); 11.13 (1H, s, OH). ¹³C NMR (CDCl₃, δ): 12.5 (CH₃), 20.0 (CH₃), 105.3, 109.5 (Pyrrol), 117.0, 119.4, 126.2, 127.5, 128.1, 128.6, 130.5, 130.7, 131.7, 132.4, 132.6, 136.9 (Phenyl, Pyrrol), 157.7 (Imin), 158.8 (Phenol).
Synthese der Beta(pyrrolimino)enolat-Ti-/Zr-Komplexe 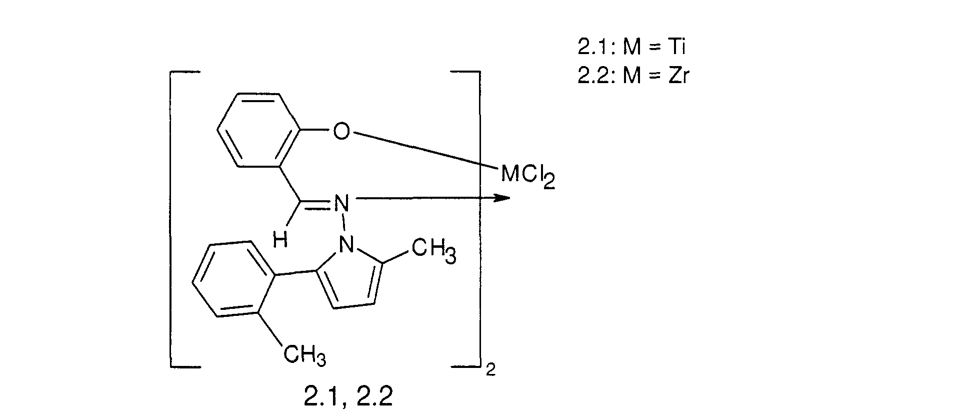
Ti-Komplex 2.1

Zu einer Lösung von 0.486 g (1,67 mmol) des Liganden 1 in 60 ml Diethylether wurden bei -78 °C 0,75 ml einer 1,6 molaren n-Butyllithium-Lösung in Hexan gegeben. Man ließ auf Zimmertemperatur erwärmen und rührte für 1 h. Dieses Lösung wurde dann langsam zu einer auf -78°C temperierten Lösung von 0,066 ml Titantetrachlorid in 40 ml Diethylether gegeben. Das Reaktionsgemisch ließ man auf Zimmertemperatur erwärmen und rührte über Nacht; danach wurde über Celite filtriert, die Lösung eingeengt und bei 0°C ausgefällt. Man erhielt 0,3 g (0,34 mmol = 40 %) des Komplexes 2.1.

### Zr-Komplex 2.2

Zu einer Lösung von 0.2323 g (0,58 mmol) des Liganden 1 in 60 ml Diethylether wurden bei -78 °C 0,39 ml einer 1,6 molaren n-Butyllithium-Lösung in Hexan gegeben. Man ließ auf Zimmertemperatur erwärmen und rührte für 1 h. Danach wurde die Lösung auf -78 °C abgekühlt und 73 mg Zirkontetrachlorid zugegeben. Das Reaktionsgemisch ließ man auf Zimmertemperatur erwärmen und rührte über Nacht; danach wurde filtriert, das Lösungsmittel bei vermindertem Druck bis zu einem Volumen von ca. 10 ml entfernt und 15 ml Hexan zugegeben. Der ausgefallene Niederschlag wurde 3 mal mit je 15 ml Hexan gewaschen. Man erhielt 70 mg (0,07 mmol = 25 %) des Komplexes 2.2.

### Standardpolymerisationsverfahren

In einem inertisierten Kolben mit mechanischem Rührer und Ethylen-Einleitungrohr wurden 150 ml (bzw. 250 ml) Toluol vorgelegt. Dazu wurden so viele ml einer 30%iger Lösung von MAO in Toluol gegeben, daß man bezogen auf den später zugesetzten Metallkomplex 100 Äquivalente einsetzte. Im Falle von Copolymerisationen wurden 12,5 ml (bzw. 25 ml) 1-Hexen dazugegeben. Dann wurden 50 µmol (bzw. 100 µmol) des zu untersuchenden Komplexes dazugegeben. Es wurde ein Ethylenstrom durch die Reaktionslösung von 40 l/h und eine Temperatur von 30°C eingestellt. Nach 1 h wurde die Reaktion durch Zugabe eines Gemisches aus 15 ml konzentrierter Salzsäure und 50 ml Methanol gestoppt.

Der Rückstand wurde gewaschen und anschließend unter vermindertem Druck getrocknet. Einzelheiten zu den Polymerisationen sind Tabelle 1 zu entnehmen.

**Tabelle 1:**

| Polymerisationen mit den erfindungsgemäßen Beispielen | | | |
|---|---|---|---|
| Komplex | Ansatzgröße (mmol) | Ausbeute PE(g) | eta-Wert (dl/g) |
| 2.1 | 50 | 0,55 | 23,7 |
| 2.2 | 31 | 0,1 | n.b. |

## Patentansprüche

1. Komplexverbindungen der allgemeinen Formel I a und b, bei denen die Variablen wie folgt definiert sind: 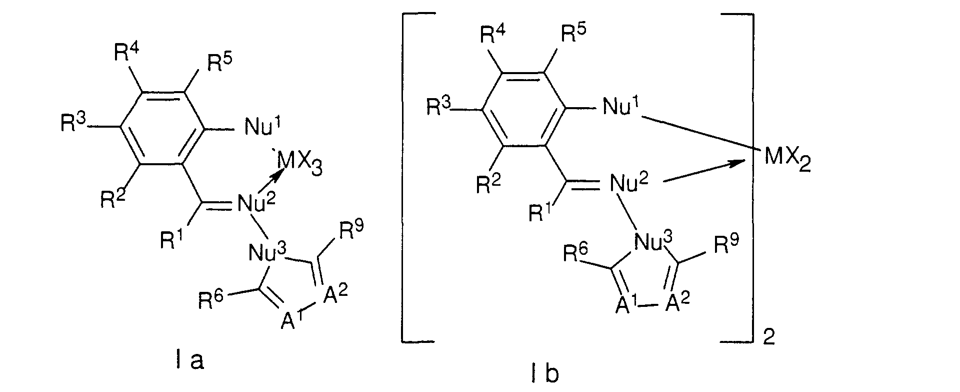
Nu¹ ausgewählt aus O, S oder Se;
Nu²,Nu³ ausgewählt aus N oder P,
M ausgewählt aus Ti, Zr, Hf oder V,
X gleich oder verschieden und ausgewählt aus Halogen, C₁-C₈-Alkyl, C₃-C₁₂-Cycloalkyl, C₇-C₁₃-Aralkyl oder C₆-C₁₄-Aryl,
A¹ N oder C-R⁷ oder Si-R⁷,
A² N oder C-R⁸ oder Si-R⁸,
R¹ bis R⁹ gleich oder verschieden und ausgewählt aus Wasserstoff,
C₁-C₈-Alkyl, substituiert oder unsubstituiert,
C₂-C₈-Alkenyl, substituiert oder unsubstituiert, mit ein bis 4 isolierten oder konjugierten Doppelbindungen;
C₃-C₁₂-Cycloalkyl, substituiert oder unsubstituiert,
C₇-C₁₃-Aralkyl,
C₆-C₁₄-Aryl, unsubstituiert oder einfach oder mehrfach gleich oder verschieden substituiert mit
C₁-C₈-Alkyl, substituiert oder unsubstituiert,
C₃-C₁₂-Cycloalkyl,
C₇-C₁₃-Aralkyl,
C₆-C₁₄-Aryl,
Halogen,
C₁-C₆-Alkoxy, substituiert oder unsubstituiert,
C₆-C₁₄-Aryloxy,
SiR¹⁰R¹¹R¹² oder O-SiR¹⁰R¹¹R¹²;
fünf- bis sechsgliedrigen stickstoffhaltigen Heteroarylresten, unsubstituiert oder einfach oder mehrfach gleich oder verschieden substituiert mit
C₁-C₈-Alkyl, substituiert oder unsubstituiert,
C₃-C₁₂-Cycloalkyl,
C₇-C₁₃-Aralkyl,
C₆-C₁₄-Aryl,
Halogen,
C₁-C₆-Alkoxy,
C₆-C₁₄-Aryloxy,
SiR¹⁰R¹¹R¹² oder O-SiR¹⁰R¹¹R¹²;
wobei räumlich benachbarte Reste R¹ bis R⁹ miteinander zu einem 5- bis 12-gliedrigen Ring verbunden sein können;
R¹⁰ bis R¹² gleich oder verschieden und ausgewählt werden aus Wasserstoff, C₁-C₈-Alkyl, C₃-C₁₂-Cycloalkyl, C₇-C₁₃-Aralkyl oder C₆-C₁₄-Aryl.

2. Komplexverbindungen nach Anspruch 1, bei denen die Variablen wie folgt definiert sind:
Nu¹ ist Sauerstoff,
Nu²,Nu³ sind Stickstoff,
A¹ N oder C-R⁸,
A² N oder C-R⁹,
R¹ Wasserstoff,
C₁-C₈-Alkyl, substituiert oder unsubstituiert, C₃-C₁₂-Cycloalkyl, substituiert oder unsubstituiert, C₇-C₁₃-Aralkyl oder
C₆-C₁₄-Aryl, unsubstituiert oder einfach oder mehrfach gleich oder verschieden substituiert mit
C₁-C₈-Alkyl, substituiert oder unsubstituiert,
R² bis R⁹ sind gleich oder verschieden und ausgewählt aus
Wasserstoff,
C₁-C₈-Alkyl, substituiert oder unsubstituiert,
C₂-C₈-Alkenyl, substituiert oder unsubstituiert, mit ein bis 4 isolierten oder konjugierten Doppelbindungen;
C₃-C₁₂-Cycloalkyl, substituiert oder unsubstituiert,
C₇-C₁₃-Aralkyl,
C₆-C₁₄-Aryl, unsubstituiert oder einfach oder mehrfach gleich oder verschieden substituiert mit
C₁-C₈-Alkyl, substituiert oder unsubstituiert,
Halogen,
C₁-C₆-Alkoxy,
wobei räumlich benachbarte Reste R¹ bis R⁹ miteinander zu 5-bis 12-gliedrigen Ring verbunden sein können.

3. Komplexverbindungen nach den Ansprüchen 1 und 2, bei denen die Variablen wie folgt definiert sind:
R¹,R²,R⁴ Wasserstoff,
R³,R⁵ unabhängig voneinander Wasserstoff oder C₁-C₈-Alkyl, verzweigt oder unverzweigt,
R⁶,R⁹ C₁-C₈-Alkyl, gleich oder verschieden, verzweigt oder unverzweigt.

4. Verfahren zur Herstellung von Komplexverbindungen gemäß den Ansprüchen 1 bis 3, **dadurch gekennzeichnet, dass** man einen Liganden der allgemeinen Formel II 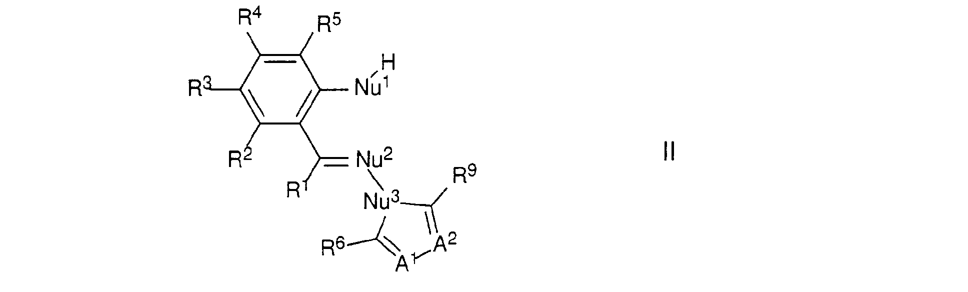 zunächst mit Hilfe einer Base deprotoniert und anschließend mit einer Metallverbindung MX₄ umsetzt, wobei M ausgewählt ist aus Ti, Zr, Hf oder V und X Halogen, C₁-C₈-Alkyl, C₃-C₁₂-Cycloalkyl, C₇-C₁₃-Aralkyl oder C₆-C₁₄-Aryl bedeutet und wobei MX₄ optional durch Neutralliganden stabilisiert werden kann.

5. Verfahren zur Polymerisation oder Copolymerisation von Olefinen unter Verwendung von Komplexverbindungen gemäß den Ansprüchen 1 bis 3.

6. Verfahren zur Herstellung eines Trägerkatalysators für die Polymerisation oder Copolymerisation von Olefinen, **dadurch gekennzeichnet, dass** man eine oder mehrere Komplexverbindungen nach Anspruch 1 bis 3 und optional einen Aktivator auf einem festen Träger abscheidet.

7. Trägerkatalysator für die Polymerisation oder Copolymerisation von Olefinen gemäß Anspruch 6.

8. Verfahren zur Polymerisation oder Copolymerisation von Olefinen unter Verwendung eines Trägerkatalysators gemäß Anspruch 7.
